# EUROPÄISCHE PATENTSCHRIFT

(11) **EP 4 061 778 B1**
(45) Veröffentlichungstag und Bekanntmachung des Hinweises auf die Patenterteilung: **02.04.2025**
(21) Anmeldenummer: 20811289.6
(22) Anmeldetag: 20.11.2020
(51) Int. Cl.: C03B 5/03, C03B 5/027, C03C 3/083, C03B 5/43, C03C 3/087

(54) **GLAS, VERFAHREN ZUR HERSTELLUNG EINES GLASES UND GLASSCHMELZANLAGE**
GLASS, METHOD OF PRODUCING GLASS AND GLASS MELTING FURNACE
VERRE, PROCÉDÉ DE PRODUCTION DE VERRE ET FOUR DE FUSION DE VERRE

(30) Priorität: 21.11.2019 DE 102019217977
(43) Veröffentlichungstag der Anmeldung: 28.09.2022
(73) Patentinhaber: SCHOTT AG, 55122 Mainz (DE)
(72) Erfinder: VOGL, Armin, 07751 Jena (DE); HESSENKEMPER, Joerg, 07749 Jena (DE); JAKOBI, Knut, 07743 Jena (DE); DUCH, Klaus-Dieter, 65232 Taunusstein (DE); SPRENGER, Andreas, 07751 Rothenstein (DE); HAHN, Michael, 65329 Hohenstein (DE); KOENIG, Klaus-Peter, 07745 Jena (DE); PFEIFFER, Thomas, 55218 Ingelheim (DE)
(74) Vertreter: Ullrich & Naumann PartG mbB
(86) Internationale Anmeldenummer: PCT/EP2020/082860
(87) Internationale Veröffentlichungsnummer: WO 2021/099553

(56) Entgegenhaltungen:
- EP-A1- 2 789 587
- EP-A1- 3 239 110
- EP-A1- 3 553 034
- US-A1- 2006 144 089
- US-B2- 6 829 908

## Beschreibung

Die Erfindung betrifft ein Glaselement aus Borosilikatglas.

Die Erfindung betrifft weiter ein Verfahren zur Herstellung eines Glaselements aus Borosilikatglas.

Die Erfindung betrifft darüber hinaus eine Glasschmelzanlage, sowie ein Verfahren zum Betreiben einer Glasschmelzanlage.

Obwohl die vorliegende Erfindung allgemein auf beliebige Glaselemente anwendbar ist, wird die vorliegende Erfindung in Bezug auf ein Glaselement in Form einer Glasplatte erläutert.

Glasschmelzanlagen zur Herstellung von Glas bestehen in der Regel aus einem Einschmelz- oder Erwärmbereich, in welchem aus den Rohstoffen, dem sogenannten Glasgemenge, eine flüssige Glasschmelze gebildet wird, aus einem nachfolgenden Läuterbereich, in welchem die Glasschmelze geläutert wird, so dass die nach dem Einschmelz- bzw. Erwärmprozess noch verbliebenen Restgasblasen aus der Glasschmelze entfernt werden, und aus einem nachgeordneten Konditionierungsbereich, welcher der weiteren Konditionierung der ausgeläuterten Glasschmelze dient. Die für den gesamten Schmelzprozess notwendigen Temperaturen sind dabei stark von der Glasart abhängig. So werden Kalk-Natron-Gläser, beispielsweise zur Produktion von Fensterglas und Glasbehältern, bei deutlich niedrigeren Temperaturen geschmolzen als beispielsweise Spezialgläser für Displayanwendungen oder auch Glaskeramiken. Der Konditionierungsbereich einer derartigen Glasschmelzanlage kann dabei als Arbeitswanne oder auch als Rinnen- und Verteilersystem ausgebildet sein. Üblicherweise wird als Wandmaterial einer Glasschmelzanlage ein Feuerfestmaterial verwendet, zumeist bestehend aus Aluminium-Zirkonium-Silikat-Material. Es ist jedoch bekannt, dass die Verwendung derartiger Materialien als Wandmaterial oder Schmelzkontaktmaterial zur Bildung von Blasen und/oder Schlieren in der Glasschmelze und letztendlich zu Ausschuss im Glasendprodukt führen können.

Auch weitere Defekte, beispielsweise aus dem Wandmaterial der Schmelzwanne herausgelöste Partikel oder dergleichen führen zu entsprechenden Einschlüssen im Mikrometerbereich, beispielsweise im gefloatetem Borosilikatglas, welches dann dieses Glas für verschiedene Anwendungen ungeeignet macht. In bekannter Weise hergestellte Proben von Glasplatten aus gefloatetem Borosilikatglas weisen störende metallische und nicht-metallische Einschlüsse und Blasen mit einer breiten Größenverteilung und hoher Anzahl auf. Einschlüsse mit einer Größe von mehr als 50 µm können mittels herkömmlichen optischen Detektionsverfahren aussortiert werden. Hingegen können Einschlüsse mit einer Größe von 50 µm oder weniger nur sehr schwer detektiert werden und können meist nur sehr aufwendig durch geschultes Personen identifiziert, quantifiziert und qualifiziert werden.

Bekannte Glaselemente und Verfahren zu deren Herstellung sind beispielsweise aus der US 6,829,908 B2 oder der US 2006/144089 A1 bekannt.

Eine Aufgabe der vorliegenden Erfindung ist es daher, ein Glaselement, bevorzugt eine Glasplatte, mit reduzierter Anzahl an Einschlüssen im Mikrometerbereich bereitzustellen und ein Verfahren zur Herstellung des Glaselements, bevorzugt einer Glasplatte, eine Glasschmelzanlage, und ein Verfahren zum Betreiben der Glasschmelzanlage anzugeben, welches die Anzahl von Einschlüssen im Mikrometerbereich erheblich reduziert.

Eine weitere Aufgabe der vorliegenden Erfindung ist es, ein alternatives Glaselement, bevorzugt eine Glasplatte, ein alternatives Verfahren zur Herstellung eines Glaselements, bevorzugt eine Glasplatte, eine alternative Glasschmelzanlage, und ein alternatives Verfahren zum Betreiben der Glasschmelzanlage anzugeben.

Die vorliegende Erfindung löst die vorstehend genannten Aufgaben mit einem Glaselement gemäß Anspruch 1.

Die vorliegende Erfindung löst die vorstehend genannten Aufgaben mit einem Verfahren zur Herstellung eines Glaselements gemäß Anspruch 11.

Die vorliegende Erfindung löst die vorstehend genannten Aufgaben mit einer Glasschmelzanlage gemäß Anspruch 13.

Die vorliegende Erfindung löst die vorstehend genannten Aufgaben ebenfalls mit einem Verfahren zum Betreiben einer Glasschmelzanlage gemäß Anspruch 14.

Darüber hinaus offenbart die vorliegende Beschreibung ebenfalls ein Glaselement, bevorzugt Glaselement aus Borosilikatglas, bevorzugt in Form einer Borosilikatglasplatte, bevorzugt gemäß einem der Ansprüche 1 bis 10, hergestellt mit einem Verfahren gemäß einem der Ansprüche 11 bis 12 und/oder mittels einer Glasschmelzanlage gemäß Anspruch 13.

Unter dem Begriff Zirkoniumoxid ist hierin ZrO₂ zu verstehen.

Falls nicht anders definiert, sind hierin % gleich Gew.-%.

Einer der damit erzielten Vorteile ist, dass damit Einschlüsse, d.h. metallische und nicht-metallische Einschlüsse sowie Blasen, erheblich reduziert werden können. Bevorzugt bei einer Durchführung der Schritte ii) bis iii) und wenn alle Bedingungen a) und c) bzw. A) und C) erfüllt bzw. die Schritte I) bis III) durchgeführt werden, kann sowohl die Anzahl von metallischen Einschlüssen, als auch die Anzahl von nicht-metallischen Einschlüssen, insbesondere von Feuerfestmaterial und von Rekristallisationsprodukten von im Glas gelösten Korrosionsprodukten, erheblich gesenkt werden. Hierbei ist erfindungsgemäß erkannt worden, dass bevorzugt bei Erfüllung der Bedingung a) bzw. A) bzw. Durchführung von Schritt I) die Anzahl von metallischen Einschlüssen reduziert wird. Wird der erfindungsgemäße Frequenzbereich unterschritten, ist erkannt worden, dass überraschenderweise die Anzahl von metallischen Einschlüssen angestiegen ist, wohingegen eine Erhöhung der Frequenz über den vorgenannten Frequenzbereich hinaus nur äußerst aufwendig und damit kostenintensiv implementiert werden kann. Durch Schritt II), ist erfindungsgemäß erkannt worden, dass dies eine Reduzierung von nicht-metallischen Einschlüssen von Feuerfestmaterial ermöglicht. Wird Bedingung c) bzw. C) erfüllt bzw. Schritt III) durchgeführt, ist erfindungsgemäß erkannt worden, dass dies zur Reduzierung von Einschlüssen von Rekristallisationsprodukten von im Glas gelösten Korrosionsprodukten führt.

Ausführungsformen sind jene, bei denen Bedingung(en) bzw. Schritt(e) a, A bzw. I und II; c, C bzw. II und III; oder a, A bzw. I und II und c, C bzw. III; erfüllt sind bzw. durchgeführt werden.

Gemäß einer bevorzugten Ausführungsform ist das Glaselement eine Glasplatte; eine pharmazeutische Primärverpackung aus Glas, bevorzugt ein Glasvial, eine Glasspritze, eine Glasampulle und/oder eine Glaskarpule; oder ein Glasrohr, bevorzugt hergestellt aus Borosilikatglas.

Unter dem Begriff "Bodenmaterial" in Bezug auf den Begriff "Glasschmelze" ist jener Materialbereich der Glasschmelze zu verstehen, der durch Öffnungen im Boden, beispielsweise durch eine Öffnung in einer Wanne der Erwärmeinrichtung und/oder der Konditionierungseinrichtung, bevorzugt der Konditionierungseinrichtung, abgezogen werden kann. Hierin bedeutet allgemein Abzug eines bestimmten % des Volumenstroms von dem Bodenmaterial der Glasschmelze in einem Bodenbereich der Glasschmelze, dass ein bestimmter Vol.-%-Anteil der Glasschmelze im Bodenbereich pro Zeiteinheit abgezogen wird. Nur der überstehende Teil der Glasschmelze dient zur Ausbildung des Glaselements, bevorzugt in Form einer Glasplatte. Der Abzug aus der Öffnung/den Öffnungen im Boden und der Abzug des überstehenden Teils der Glasschmelze ergibt insgesamt 100 % des Volumenstroms. Wird z.B. innerhalb eines Zeitintervalls 25 Liter von dem Bodenmaterial der Glasschmelze in einem Bodenbereich der Glasschmelze abgezogen und werden innerhalb desselben Zeitintervalls 75 Liter der Glasschmelze zum Ausbilden des Glaselements, bevorzugt der Glasplatte abgezogen, so entspricht dies einem Abzug von 25 % des Volumenstroms von dem Bodenmaterial der Glasschmelze in einem Bodenbereich der Glasschmelze.

Unter dem Begriff "Kontaktfläche" in Bezug auf den Begriff "Glasschmelze" ist diejenige Fläche zu verstehen, die mit schmelzflüssigem Glas in Kontakt ist bzw. steht. Dies umfasst zum Beispiel die Innenwand der Schmelzwanne, auch Arbeitswanne genannt, bei üblichem Befüllungsgrad, Rührer, Elektrodenhalterungen und Elektroden, Ein- und Auslässe der jeweiligen Wannen(abschnitte), Rinnen innerhalb der Schmelzwanne und dergleichen. Nicht zur Kontaktfläche zählen hierin insbesondere Flächen einer Zuführeinrichtung des Gemisches in die Schmelzwanne und Kontaktflächen nach den direkten Auslässen aus der Schmelzwanne, z.B. das Floatbad und/oder der Kühlkanal.

Unter dem Begriff "Glaselement" ist ein beliebig geformtes dreidimensionales Objekt zu verstehen, welches zumindest teilweise, bevorzugt überwiegend, vorzugsweise vollständig aus Glas, mehr bevorzugt Borosilikatglas, hergestellt ist und bevorzugt als Glasplatte; als pharmazeutische Primärverpackung aus Glas, bevorzugt als Glasvial, als Glasspritze, als Glasampulle und/oder als Glaskarpule; oder als Glasrohr ausgebildet sein kann.

Unter dem Begriff "Glasplatte" ist ein dreidimensionales Objekt aus Glas zu verstehen, welches beispielsweise im einfachsten Fall im Wesentlichen als ein Quader mit einer Dicke, einer Länge und einer Breite ausgebildet ist. Die kleinste Dimension ist die Dicke, die beiden größten Dimensionen stellen eine Fläche mit einer Länge und einer Breite dar. Die Betrachtungsebene ist entlang einer Normalen der Fläche.

Dicke, Länge und Breite sind frei wählbar. Bevorzugt beträgt die Dicke zwischen 0,01 cm und 10 cm, mehr bevorzugt zwischen 0,1 cm und 5 cm, mehr bevorzugt zwischen 0,15 cm und 2,5 cm, meist bevorzugt zwischen 0,2 und 0,4 cm. Bevorzugt beträgt die Länge und Breite jeweils zwischen 1 cm und 500 cm, mehr bevorzugt zwischen 3 cm und 400 cm, mehr bevorzugt zwischen 5 und 100 cm, mehr bevorzugt zwischen 20 und 70 cm, meist bevorzugt 40 cm bis 60 cm. Vorteil hiervon ist die Bereitstellung einer dünnen durchsichtigen Glasplatte, beispielsweise als durchsichtige Abdeckung oder dergleichen.

Unter dem Begriff "Glasrohr" ist ein dreidimensionales Objekt aus Glas zu verstehen, welches beispielsweise im einfachsten Fall im Wesentlichen als ein länglicher Hohlkörper mit einem Außendurchmesser, einer Länge und einer Wandstärke ausgebildet ist. Die kleinste Dimension ist beispielsweise die Wandstärke, die beiden größeren Dimensionen sind Außendurchmesser und Länge. Die Betrachtungsebene ist entlang einer Normalen der äußeren Fläche des Rohrs.

Außendurchmesser, Länge und Wandstärke sind frei wählbar. Bevorzugt beträgt die Länge des Glasrohrs 2 cm oder mehr, bevorzugt 10 cm oder mehr, mehr bevorzugt 20 cm oder mehr, mehr bevorzugt 30 cm oder mehr, mehr bevorzugt 40 cm oder mehr, mehr bevorzugt 50 cm oder mehr, mehr bevorzugt 110 cm oder mehr und/oder 500 cm oder weniger, bevorzugt 400 cm oder weniger, mehr bevorzugt 300 cm oder weniger, mehr bevorzugt 200 cm oder weniger, mehr bevorzugt 100 cm oder weniger, mehr bevorzugt 50 cm oder weniger. Bevorzugt beträgt der Außendurchmesser 3 mm oder mehr, bevorzugt 4 mm oder mehr, mehr bevorzugt 5 mm oder mehr, mehr bevorzugt 6 mm oder mehr, mehr bevorzugt 7 mm oder mehr, mehr bevorzugt 8 mm oder mehr, mehr bevorzugt 9 mm oder mehr, mehr bevorzugt 10 mm oder mehr, mehr bevorzugt 15 mm oder mehr, mehr bevorzugt 20 mm oder mehr und/oder 20 cm oder weniger, bevorzugt 15 cm oder weniger, mehr bevorzugt 10 cm oder weniger, mehr bevorzugt 5 cm oder weniger, mehr bevorzugt 4 cm oder weniger, mehr bevorzugt 3 cm oder weniger, mehr bevorzugt 2 cm oder weniger. Bevorzugt beträgt die Wandstärke 0,1 mm, bevorzugt 0,5 mm, mehr bevorzugt 0,8 mm, mehr bevorzugt 1,0 mm, mehr bevorzugt 1,5 mm, mehr bevorzugt 2,0 mm, mehr bevorzugt 3,0 mm und/oder 10,0 mm oder weniger, bevorzugt 5,0 mm oder weniger, mehr bevorzugt 5,0 mm oder weniger, mehr bevorzugt 4,0 mm oder weniger, mehr bevorzugt 3,0 mm oder weniger, mehr bevorzugt 2,0 mm oder weniger, mehr bevorzugt 1,0 mm oder weniger.

Die Transmission des Glaselements ist nicht auf spezielle Weise beschränkt. Bevorzugt ist die Transmission des Glaselements normiert auf ein Glaselement, bevorzugt auf eine Glasplatte oder Glasrohr, mit einer Dicke oder Wandstärke von 6,5 mm bei einer Wellenlänge von 400 nm bis 800 nm 70 % oder mehr, mehr bevorzugt 80% oder mehr, mehr bevorzugt 90 % oder mehr, meist bevorzugt 95 % oder mehr.

Die Transmission wird üblicherweise mit einem UV-VIS-Spektrometer, z.B. Spektralphotometer Specord 250 Plus (Fa. Analytik Jena), gemessen. Gemessen wird die Transmission der Probe im Wellenlängenbereich von 250 bis 1050 nm; die Auswertung der Messung erfolgt üblicherweise nach DIN EN 410:2011-04 (Glas im Bauwesen - Bestimmung der lichttechnischen und strahlungsphysikalischen Kenngrößen von Verglasungen; Deutsche Fassung EN 410:2011).

Die Bestimmung der Anzahl der Einschlüsse und der Größe der Einschlüsse gemäß Ausführungsformen der Erfindung wurde mittels des im folgenden beschriebenen Verfahrens durchgeführt:
In ein zu untersuchendes Glaselement, hier eine Glasplatte, wird in einem Dunkelraum sichtbares Licht senkrecht zur Erstreckung der Dicke der Glasplatte, bevorzugt an einer geraden Schnittkante, und parallel zu der Fläche, die durch die beiden größten Dimensionen (Länge und Breite) gebildet wird, eingekoppelt. Durch die Reflexion des Lichts an den Einschlüssen in der Glasplatte werden die Einschlüsse identifiziert. Für die Unterscheidung zwischen Staub und Einschlüssen in der Glasplatte kleiner Partikel, etwa im Größenbereich < 20 µm, kann ein Handmikroskop, beispielsweise ein Handmikroskop "Wide Stand Microscope" der Firma PEAK verwendet werden. Die so optisch identifizierten Partikel werden sichtbar markiert. Die so markierte Glasplatte wird entlang der Normalen der Fläche unter einem Lichtmikroskop, beispielsweise dem Axio Imager M2m der Firma Zeiss, mit Objektiv LD EC Epiplan 50x/0,55 HD DIC und Okular PI 10x/2 betrachtet, wodurch die Einschlüsse klassifiziert werden und die Größe der Einschlüsse vermessen wird. Die Größe der Einschlüsse und Partikel bezieht sich hierbei auf die längste in der Betrachtungsebene sichtbaren Dimension. Bei dieser Art der Messung wird bewusst in Kauf genommen, dass dreidimensional ausgebildete Einschlüsse sich mit deren maximaler Längserstreckung bei der Messung auch in Richtung der optischen Achse des Mikroskops erstrecken können, d.h. entlang der Normalen der Betrachtungsebene, und in diesem Falle dann ein kleinerer gemessener Wert des Einschlusses erhalten wird als der tatsächlich Wert der vorliegenden Längserstreckung des Einschlusses, beispielsweise des Kristalls oder Kristallits. Die minimale Größe der Einschlüsse, die mit dem Axio Imager M2m der Firma Zeiss messbar sind beziehungsweise gemessen werden können, beträgt circa 2 µm. Kleinere Einschlüsse unterhalb einer Größe von 2 µm werden dann entsprechend nicht bei der Zählung der Einschlüsse berücksichtigt.

Auch wenn nicht im Detail beschrieben, hat ein Fachmann keine Probleme das oben beschriebene Verfahren auf ein Glaselement, wie z.B. ein Glasrohr, anzuwenden.

Kleinere Einschlüsse unterhalb einer Größe von 2 µm bis hinunter zu einer Größe von einschließlich 50 nm können beispielsweise mittels des Röntgen-Mikroskops "Xradia 800 Ultra" der Firma Zeiss gemessen werden. Noch kleinere Einschlüsse mit einer Größe von weniger als 50 nm werden entsprechend nicht im Sinne eines Einschlusses gezählt beziehungsweise berücksichtigt.

Wiegt ein Glaselement weniger als ein Kilogramm, so berechnen sich die Einschlüsse anteilig. Wiegt das Glaselement mehr als ein Kilogramm, so wird die Anzahl der Einschlüsse entsprechend multipliziert. Das Gewicht des Glaselements ist nicht besonders beschränkt. Das Glaselement wiegt bevorzugt 5 g bis 400 kg, mehr bevorzugt 0,01 kg bis 350 kg, mehr bevorzugt 0,1 kg bis 20 kg, mehr bevorzugt 0,2 kg bis 18 kg, mehr bevorzugt 0,5 kg bis 15 kg, meist bevorzugt 1,0 kg bis 10 kg.

Soweit nicht anders angegeben, sind hierin alle %-Angaben als Gewichtsprozent, abgekürzt Gew.-%, zu verstehen.

Der Begriff "Einschluss" ist hierin im weitesten Sinne zu verstehen und umfasst insbesondere Einschlüsse in Form von metallischen und nicht-metallischen Einschlüssen, auch Partikel genannt, und in Form von Gas(en), z.B. Luft, also Blasen.

Der Begriff "metallischer Einschluss" ist hierin im weitesten Sinne zu verstehen. Metallische Einschlüsse sind hierin insbesondere Einschlüsse von Partikeln aus einem oder mehreren überwiegend, elementar vorliegenden Metall(en). Das Metall / Die Metalle der metallischen Einschlüsse hat / haben somit die Oxidationsstufe gleich 0. Metallische Einschlüsse umfassen insbesondere Edelmetalle und Refraktärmetalle.

Der Begriff "nicht-metallischer Einschluss" ist im weitesten Sinne zu verstehen. Nicht-metallische Einschlüsse sind hierin Einschlüsse bestehend aus Kationen (Oxidationsstufe >0) und Anionen (Oxidationsstufe <0), auch Salze genannt. Insbesondere umfassen nicht-metallische Einschlüsse Partikel in Form von Kristallen aus Glasbestandteilen und einem oder mehreren Salzen von Refraktärmetallen und Edelmetallen, im speziellen Kristallen aus Glasbestandteilen und ein oder mehrere Silikate oder Oxide von Refraktärmetallen und Edelmetallen.

Hierin werden Einschlüsse als metallische Einschlüsse gezählt, wenn sie im Wesentlichen aus elementarem Metall, also aus circa 100% elementarem Metall (Oxidationsstufe gleich 0) bestehen, ansonsten werden die Einschlüsse als nicht-metallische Einschlüsse gezählt. Eine Unterscheidung zwischen metallisch und nicht-metallisch kann auf optischem Weg, beispielsweise wir oben beschrieben, erfolgen. Refraktärmetalle sind zum Beispiel die Metalle Titan, Zirkonium, Hafnium, Vanadium, Niob, Tantal, Chrom, Molybdän und Wolfram.

Edelmetalle umfassen hierin Halbedelmetalle und sind zum Beispiel Ruthenium, Rhodium, Palladium, Osmium, Iridium, Platin, Silber, Gold, Kupfer, Technetium, Rhenium, Antimon, Bismut und Polonium.

Weitere Beispiele von Metallen, die in den Einschlüssen enthalten sein können, sind Magnesium, Kalzium, Aluminium, Silizium, Zinn (metallische Einschlüsse) und Salze davon (nicht-metallische Einschlüsse).

Der Begriff "Blasen" ist im weitesten Sinne zu verstehen. Blasen werden durch Gaseinschlüsse im Glas gebildet, wobei das Gas sowohl bei Raumtemperatur (20°C) gasförmig sein kann, als auch nach Abkühlung kondensieren kann und eine Blase mit Unterdruck entsteht. Gase, die Einschlüsse bilden, umfassen zum Beispiel Sauerstoff, Stickstoff, Stickoxide, Kohlenstoffdioxid, Schwefeldioxid oder dergleichen.

Hierin gelten, sofern nicht anders definiert, alle bevorzugten Parameter und Ausführungsformen des Glaselements ebenfalls für die beiden Verfahren und die Glasschmelzanlage und umgekehrt.

Weitere Merkmale, Vorteile und weitere Ausführungsformen der Erfindung sind im Folgenden beschrieben oder werden dadurch offenbar.

Gemäß einer bevorzugten Weiterbildung weist das Glaselement pro kg Glas 50 Einschlüsse oder weniger mit einer Größe von 500 nm oder mehr und 10 µm oder weniger, bevorzugt mit einer Größe von 50 nm oder mehr und 10 µm oder weniger, mehr bevorzugt von 10 µm oder weniger auf. Vorteil hiervon ist, dass eine höhere Qualität des Glases bevorzugt für Hochenergielaseranwendungen bereitgestellt wird.

Gemäß einer bevorzugten Ausführungsform weist das Glaselement pro kg Glas 2000 Einschlüsse oder weniger, bevorzugt 1000 Einschlüsse oder weniger, mehr bevorzugt 500 Einschlüsse oder weniger, mehr bevorzugt 250 Einschlüsse oder weniger, mehr bevorzugt 100 Einschlüsse oder weniger, mehr bevorzugt 50 Einschlüsse oder weniger, mit einer Größe von 20 µm oder weniger, bevorzugt 30 µm oder weniger, mehr bevorzugt 40 µm oder weniger, meist bevorzugt 50 µm oder weniger und einer Größe von 2 µm oder mehr, bevorzugt 50 nm oder mehr auf. Vorteil hiervon ist, dass eine höhere Qualität des Glases bevorzugt für Hochenergielaseranwendungen bereitgestellt wird. Bleibt die Anzahl von Einschlüssen gleich, variieren diese jedoch in einem größeren Größenbereich, werden Hochenergielaseranwendungen weniger beeinträchtigt.

Dabei ist folgendes zu beachten:
Ist beispielsweise die Anzahl der Einschlüsse pro kg Glas auf 50 Einschlüsse oder weniger mit einer Größe von 10 µm oder weniger beschränkt, darf ein Glaselement gemäß einer Ausführungsform der vorliegenden Erfindung pro kg Glas 50 Einschlüsse mit einer Größe von 5 µm und 50 Einschlüsse mit einer Größe von 40 µm aufweisen. Ist jedoch die Anzahl der Einschlüsse pro kg Glas auf 50 Einschlüsse oder weniger mit einer Größe von 50 µm oder weniger beschränkt, so würde ein Glaselement, das beispielsweise pro kg Glas 50 Einschlüsse mit einer Größe von 5 µm und 50 Einschlüsse mit einer Größe von 40 µm aufweist, nicht diese Bedingung erfüllen, jedoch aber ein Glaselement, das beispielsweise 10 Einschlüsse mit einer Größe von 5 µm, 20 Einschlüsse mit einer Größe von 25 µm und 15 Einschlüsse mit einer Größe mit einer Größe von 45 µm aufweist.

Das Glaselement kann beliebig viele weitere größere Einschlüsse aufweisen. Große Einschlüsse können leicht mittels üblicher Messtechnik beispielsweise direkt nach der Produktion des Glaselements und/oder vor Weiterverarbeitung detektiert werden und das Glaselement ganz oder teilweise aussortiert werden. Mit den hierin beschriebenen Ausführungsformen der Erfindung können ebenfalls größere Einschlüsse deutlich reduziert werden. Daher weist das Glaselement bevorzugt keine Einschlüsse mit einer Größe von mehr als 50 µm, mehr bevorzugt mehr als 40 µm, mehr bevorzugt mehr als 30 µm, mehr bevorzugt mehr als 20 µm, meist bevorzugt mehr als 10 µm, auf.

Gemäß einer bevorzugten Ausführungsform weist das Glaselement pro kg Glas 50 Einschlüsse oder weniger, bevorzugt 40 Einschlüsse oder weniger, bevorzugt 30 Einschlüsse oder weniger, mehr bevorzugt 20 Einschlüsse oder weniger, meist bevorzugt 10 Einschlüsse oder weniger, auf, wobei diese eine Größe von 10 µm oder weniger, bevorzugt 20 µm oder weniger, bevorzugt 30 µm oder weniger, mehr bevorzugt 40 µm oder weniger, meist bevorzugt 50 µm oder weniger, aufweisen können. Vorteil hiervon ist, dass die Qualität des Glaselements, bevorzugt für Hochenergielaseranwendungen, erhöht ist. Je weniger Einschlüsse pro kg Glas vorhanden sind, desto weniger werden Hochenergielaseranwendungen beeinträchtigt. Die Qualität des Glaselements wird verbessert.

Je nach Variation der Parameter, also Frequenz des Wechselstroms, prozentualer Abzug des Volumenstroms und Anteil von Zirkoniumoxid in der Kontaktfläche können sowohl die Anzahl der Einschlüsse als auch deren Größe beeinflusst werden. Auch bei Erfüllung lediglich einer Bedingung a) bzw. A), b) bzw. B) und c) bzw. C) bzw. Durchführung lediglich eines Schritts I), II) oder III) wird zumindest einer der Parameter Größe der Einschlüsse und/oder Anzahl der Einschlüsse pro kg Glas reduziert.

Gemäß einer bevorzugten Ausführungsform umfassen die Einschlüsse metallische Einschlüsse, wobei das Glaselement pro kg Glas 40 metallische Einschlüsse oder weniger, bevorzugt 30 metallische Einschlüsse oder weniger, mehr bevorzugt 20 metallische Einschlüsse oder weniger, mehr bevorzugt 10 metallische Einschlüsse oder weniger, meist bevorzugt 5 metallische Einschlüsse oder weniger, mit einer Größe von 5 µm oder weniger, bevorzugt 10 µm oder weniger, mehr bevorzugt 20 µm oder weniger, mehr bevorzugt 30 µm oder weniger, mehr bevorzugt 40 µm oder weniger, meist bevorzugt 50 µm oder weniger, aufweist. Damit weist das Glaselement insgesamt wenig Einschlüsse durch metallische Partikel auf, was die Qualität des Glaselements weiter verbessert. Häufig auftretende metallische Einschlüsse umfassen insbesondere Metalle wie Wolfram, Zirkonium, Platin, Rhodium, Iridium, Molybdän, Zinn und Kupfer, da diese bei der Herstellung von Glaselementen mit dem Glas in Kontakt kommen.

Gemäß einer bevorzugten Ausführungsform umfassen die Einschlüsse nicht-metallische Einschlüsse, wobei das Glaselement pro kg Glas 25 nicht-metallische Einschlüsse oder weniger, bevorzugt 15 nicht-metallische Einschlüsse oder weniger, mehr bevorzugt 8 nicht-metallische Einschlüsse oder weniger, mehr bevorzugt 1 nicht-metallische Einschlüsse oder weniger, mehr bevorzugt 0,1 nicht-metallische Einschlüsse oder weniger, mehr bevorzugt 0,05 nicht-metallische Einschlüsse oder weniger, meist bevorzugt 0,00 nicht-metallische Einschlüsse, mit einer Größe von 2 µm oder mehr und 10 µm oder weniger, bevorzugt 2 µm oder mehr und 20 µm oder weniger, mehr bevorzugt 2 µm oder mehr und 30 µm oder weniger, mehr bevorzugt 2 µm oder mehr und 40 µm oder weniger, meist bevorzugt 2 µm oder mehr und 50 µm oder weniger aufweist. Dies reduziert sowohl Anzahl als auch Größe der nicht-metallischen Einschlüsse, was wiederum die Qualität des Glaselements erhöht. Häufig auftretende nicht-metallischen Einschlüsse umfassen insbesondere Kristalle aus Glasbestandteilen und/oder ein oder mehrere Silikate und/oder Oxide von Refraktärmetallen und/oder Edelmetallen. Die Kristalle kristallisieren z.B. aus der Glasschmelze, während sich Silikate und/oder Oxide von Refraktärmetallen und/oder Edelmetallen aus dem Kontaktmaterial, das in Kontakt mit der Glasschmelze steht, bildet.

Gemäß einer bevorzugten Ausführungsform umfassen die Einschlüsse Blasen, wobei das Glaselement pro kg Glas 25 Blasen oder weniger, bevorzugt 15 Blasen oder weniger, mehr bevorzugt 8 Blasen oder weniger, mehr bevorzugt 1 Blasen oder weniger, mehr bevorzugt 0,1 Blasen oder weniger, mehr bevorzugt 0,05 Blasen oder weniger, meist bevorzugt 0,00 Blasen, mit einer Größe von 10 µm oder weniger, bevorzugt 20 µm oder weniger, mehr bevorzugt 30 µm oder weniger, mehr bevorzugt 40 µm oder weniger, meist bevorzugt 50 µm oder weniger, und/oder 2 µm oder mehr, bevorzugt 50 nm oder mehr, aufweist. Damit ist die Qualität des Glaselements auch für Einschlüsse in Form von Blasen insgesamt erhöht.

Gemäß einer bevorzugten Ausführungsform umfasst die Zusammensetzung des Glaselements, in Gew.-%:

| | |
|---|---|
| SiO₂ | 60 bis 90 %, mehr bevorzugt 76 % bis 90 %; |
| B₂O₃ | 0 bis 20 %; |
| Al₂O₃ | 0 bis 20 %; |
| Li₂O | 0 bis 10 %; |
| Na₂O | 0 bis 10 %; |
| K₂O | 0 bis 10 %; |
| MgO | 0 bis 10 %; |
| CaO | 0 bis 10 %; |
| SrO | 0 bis 10 %; und |
| BaO | 0 bis 10 %. |

In einer weiter bevorzugten Ausführungsform weist das Glaselement folgende Zusammensetzung, in Gew.-%, auf:

| | |
|---|---|
| SiO₂ | mehr als 76 %; |
| B₂O₃ | 0 bis 15 %; |
| Al₂O₃ | 0 bis 5 %; |
| Li₂O | 0 bis 4 %; |
| Na₂O | 0 bis 4 %; |
| K₂O | 0 bis 4 %; |
| MgO | 0 bis 4 %; |
| CaO | 0 bis 4 %; |
| SrO | 0 bis 4 %; und |
| BaO | 0 bis 4 %; und |

unvermeidbare Verunreinigungen, d.h. unter 0,01 %.

In einer weiter bevorzugten Ausführungsform weist das Glaselement folgende Zusammensetzung in Gew.-%, auf:

| | |
|---|---|
| SiO₂ | 76 % bis 85 %; |
| B₂O₃ | 0 bis 15 %; |
| Al₂O₃ | 0 bis 5 %; |
| Li₂O | 0 bis 4 %; |
| Na₂O | 0 bis 4 %; |
| K₂O | 0 bis 4 %; |
| MgO | 0 bis 4 %; |
| CaO | 0 bis 4 %; |
| SrO | 0 bis 4 %; und |
| BaO | 0 bis 4 % und |

unvermeidbare Verunreinigungen, d.h. unter 0,01 %.

Bevorzugt weist das Glaselement Eisen-, Arsen- und/oder Antimonoxid mit einem Gewichtsanteil von unter 0,05 Gew.-%, mehr bevorzugt unter 0,01 Gew.-% auf. Mehr bevorzugt ist das Glaselement frei von Eisen-, Arsen- und Antimonoxid. Durch ein Arsen- und Antimonoxid-freies Glaselementwird ein umweltfreundliches Glaselement erhalten. Eisenoxid kann als Verunreinigung auftreten, was die Farbe des Glaselements beeinträchtigen kann. Durch geeignete Auswahl der Ausgangsmaterialien kann dies unterbunden werden.

Gemäß einer bevorzugten Ausführungsform weist das Glaselement ein oder mehrere der folgenden Merkmale auf:
i) das Gewicht des Glaselements beträgt 0,01 kg bis 350 kg, bevorzugt 0,1 kg bis 20 kg, mehr bevorzugt 0,5 kg bis 15 kg, meist bevorzugt 1,0 kg bis 10 kg;
ii) die Dicke des Glaselements, wenn dieses in Form einer Glasplatte bereitgestellt ist, beträgt zwischen 0,01 cm und 10 cm, bevorzugt zwischen 0,1 cm und 5 cm, mehr bevorzugt zwischen 0,15 cm und 2,5 cm, meist bevorzugt zwischen 0,2 und 0,4 cm;
iii) die Länge und Breite des Glaselements, wenn dieses in Form einer Glasplatte bereitgestellt ist, beträgt jeweils zwischen 1 cm und 500 cm, bevorzugt zwischen 3 cm und 400 cm, mehr bevorzugt zwischen 5 und 100 cm, mehr bevorzugt zwischen 20 und 70 cm, meist bevorzugt 40 cm bis 60 cm;
iv) die Transmission des Glaselements normiert auf ein Glaselement in Form einer Glasplatte mit einer Dicke von 6,5 mm bei einer Wellenlänge von 400 nm bis 800 nm beträgt 70 % oder mehr, mehr bevorzugt 80% oder mehr, mehr bevorzugt 90 % oder mehr, meist bevorzugt 95 % oder mehr; und
v) das Glaselement weist keine Einschlüsse mit einer Größe von mehr als 50 µm, bevorzugt mehr als 40 µm, mehr bevorzugt mehr als 30 µm, mehr bevorzugt mehr als 20 µm, meist bevorzugt mehr als 10 µm, auf.

Mehr bevorzugt weist das Glaselement die vorgenannten Merkmale i; ii; iii; iv; v; i+ii; i+iii; i+iv; i+v; ii+iii; ii+iv; ii+v; iii+iv; iii+v; iv+v; i+ii+iii; i+ii+iv; i+ii+v; i+iii+iv; i+iii+v; i+iv+v; ii+iii+iv; ii+iii+v; ii+iv+v; iii+iv+v; i+ii+iii+iv; i+ii+iii+v; i+ii+iv+v; i+iii+iv+v; oder i+ii+iii+iv+v auf.

Gemäß einer bevorzugten Ausführungsform des Verfahrens wird die Viskosität der Glasschmelze zumindest in den Schritten ii) und iii) auf einen Wert zwischen 30 Pas und 450 Pas, bevorzugt zwischen 33 Pas und 400 Pas, mehr bevorzugt zwischen 35 Pas und 265 Pas gebracht und/oder der Wert gehalten. Gemäß einer bevorzugten Ausführungsform des Verfahrens wird die Viskosität der Glasschmelze zumindest in Schritten ii) auf einen Wert zwischen 33 Pas und 265 Pas, bevorzugt zwischen 35 Pas und 200 Pas gebracht und/oder der Wert gehalten. Gemäß einer bevorzugten Ausführungsform des Verfahrens wird die Viskosität der Glasschmelze zumindest in Schritten iii) auf einen Wert zwischen 70 Pas und 450 Pas, bevorzugt zwischen 100 Pas und 400 Pas gebracht und/oder der Wert gehalten. Einer der damit erzielten Vorteile ist, dass das Verfahren für eine Vielzahl unterschiedlicher herzustellender Gläser mit unterschiedlichen Temperaturen während der Herstellung anwendbar ist.

Gemäß einer bevorzugten Ausführungsform ist das Glaselement in Form einer Glasplatte oder eines Glasrohrs bereitgestellt. Dies ermöglicht eine einfache Herstellung beziehungsweise flexible Nutzbarkeit des Glaselements für unterschiedlichste Anwendungen.

Gemäß einer bevorzugten Ausführungsform weist das Glaselement pro kg Glas 2000 Einschlüsse oder weniger, bevorzugt 1000 Einschlüsse oder weniger, mehr bevorzugt 500 Einschlüsse oder weniger, mehr bevorzugt 250 Einschlüsse oder weniger, mehr bevorzugt 100 Einschlüsse oder weniger, mehr bevorzugt 50 Einschlüsse oder weniger mit einer Größe von 50 nm oder mehr und 500 nm oder weniger auf. Vorteil hiervon ist, dass eine höhere Qualität des Glases bevorzugt für Hochenergielaseranwendungen bereitgestellt wird.

Gemäß einer bevorzugten Ausführungsform weist das Glaselement pro kg Glas 2000 Einschlüsse oder weniger, bevorzugt 1000 Einschlüsse oder weniger, mehr bevorzugt 500 Einschlüsse oder weniger, mehr bevorzugt 250 Einschlüsse oder weniger, mehr bevorzugt 100 Einschlüsse oder weniger, mehr bevorzugt 50 Einschlüsse oder weniger mit einer Größe von 500 nm oder mehr und 2 µm oder weniger auf. Vorteil hiervon ist eine noch höhere Qualität des Glaselements.

Gemäß einer bevorzugten Ausführungsform des Verfahrens wird als Heizstrom ein Wechselstrom mit einer Frequenz zwischen 1 kHz und 100 kHz, bevorzugt zwischen 5 kHz und 50 kHz, mehr bevorzugt zwischen 8 kHz und 15 kHz, verwendet. Ist die Frequenz 1 kHz oder mehr, bevorzugt, 5 kHz oder mehr, mehr bevorzugt 8 kHz oder mehr, wird die Anzahl der Einschlüsse, insbesondere der metallischen Einschlüsse, reduziert. Ist die Frequenz allerdings 200 kHz oder niedriger, bevorzugt 100 kHz oder niedriger, mehr bevorzugt 50 kHz oder niedriger, mehr bevorzugt 15 kHz oder niedriger, ist es einfacher diese Frequenz zu realisieren und unerwartete Probleme, wie Blasenbildung durch lokale Überhitzung, treten weniger auf.

Gemäß einer bevorzugten Ausführungsform des Verfahrens umfasst die Kontakt-fläche, mit der die Glasschmelze in Kontakt ist, 50 % oder mehr, mehr bevorzugt 60% oder mehr, mehr bevorzugt 70% oder mehr, mehr bevorzugt 80% oder mehr, mehr bevorzugt 90% oder mehr, mehr bevorzugt 95% oder mehr des Kontaktmaterials in Form von schmelzgegossenem Zirkoniumoxidmaterial. Damit wird die Anzahl der Einschlüsse beispielsweise von Feuerfestmaterial reduziert, die Qualität des Glases bzw. des Glaselements erhöht.

Gemäß einer bevorzugten Ausführungsform des Verfahrens umfasst das Kontaktmaterial schmelzgegossenes Zirkoniumoxidmaterial mit einem Anteil von über 90 Gew.-% ZrO₂, bevorzugt über 95 Gew.-% ZrO₂. Damit wird die Anzahl der Einschlüsse von Feuerfestmaterial noch weiter reduziert und die Qualität des Glaselements noch weiter erhöht.

Gemäß einer bevorzugten Ausführungsform des Verfahrens wird von dem Bodenmaterial der Glasschmelze in einem Bodenbereich der Glasschmelze zwischen 5 % und 40 %, bevorzugt zwischen 20 % und 40 %, mehr bevorzugt zwischen 25 % und 35 %, des Volumenstroms von dem Bodenmaterial der Glasschmelze in einem Bodenbereich abgezogen. Vorteil hiervon ist, dass die Anzahl der Einschlüsse von Korrosionsprodukten, d.h. nicht-metallischen Einschlüssen, reduziert und die Qualität des Glases somit erhöht wird.

In einer bevorzugten Ausführungsform werden in dem Verfahren zur Herstellung eines Glaselements in Form einer Glasplatte, bevorzugt einer Borosilikatglasplatte, die folgenden Schritte S1 bis S4; bevorzugt erst S1, dann S2, dann S3 und zuletzt S4; durchgeführt.

In einem ersten Schritt S1 erfolgt ein Bereitstellen eines Gemisches, gemäß einer Ausführungsform der vorliegenden Erfindung. In einem weiteren Schritt S2 erfolgt ein Erwärmen des Gemisches zu einer Glasschmelze. In einem weiteren Schritt S3 erfolgt ein Konditionieren der Glasschmelze, und in einem weiteren Schritt S4 erfolgt ein Abkühlen der Glasschmelze zur Bereitstellung des Glaselements.

Während der Durchführung der Schritte S2 - Erwärmen - und S3 - Konditionieren wird zumindest eine, bevorzugt alle, der folgenden Bedingungen erfüllt:
a) die Glasschmelze wird zumindest teilweise mit einer elektrischen Widerstandsbeheizung durch Beaufschlagen von Elektroden mit einem Heizstrom beheizt und/oder erwärmt, wobei als Heizstrom ein Wechselstrom mit einer Frequenz zwischen 1 kHz und 200 kHz verwendet wird;
b) die Kontaktfläche, mit der die Glasschmelze in Kontakt ist, umfasst zu 30 % oder mehr Kontaktmaterial in Form von schmelzgegossenem Zirkoniumoxidmaterial mit einem Anteil von über 70 Gew.-% ZrO₂; und
c) zwischen 1 Vol.-% und 50 Vol.-% von dem Bodenmaterial der Glasschmelze wird in einem Bodenbereich der Glasschmelze abgezogen.

Weitere bevorzugte Merkmale und Vorteile der Erfindung ergeben sich aus den Unteransprüchen, aus den Zeichnungen und der dazugehörigen Figurenbeschreibung.

Es versteht sich, dass die vorstehend genannten und die nachstehend erläuterten Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungen und Ausführungsformen der Erfindung sind in den Zeichnungen dargestellt und werden in der nachfolgenden Beschreibung näher erläutert, wobei sich gleiche Bezugszeichen auf gleiche oder ähnliche oder funktional gleiche Bauteile oder Elemente beziehen.

Dabei zeigt:
- Figur 1: eine Glasschmelzanlage gemäß einer Ausführungsform der vorliegenden Erfindung; und
- Figur 2: ein Verfahren zur Herstellung eines Glaselements gemäß einer Ausführungsform der vorliegenden Erfindung.

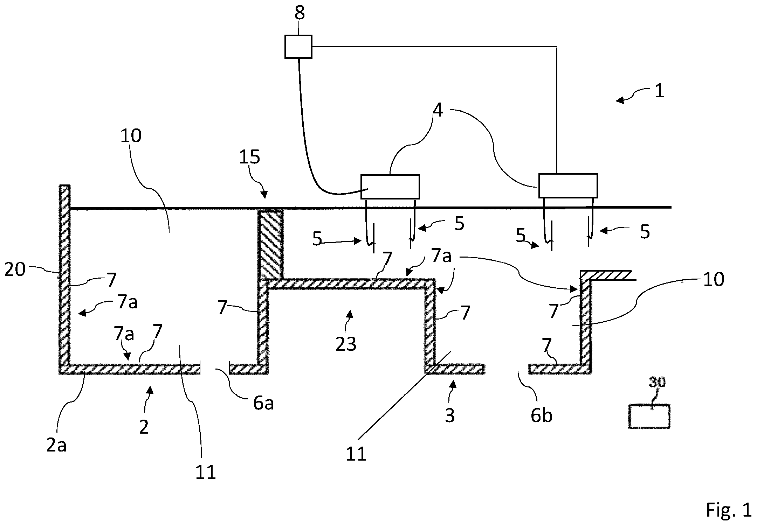

Figur 1 zeigt eine Glasschmelzanlage gemäß einer Ausführungsform der vorliegenden Erfindung.

Im Detail ist in Figur 1 eine Glasschmelzanlage 1 gezeigt. Die Glasschmelzanlage 1 umfasst eine Erwärmeinrichtung 2, in der das Erwärmen und Einschmelzen von Ausgangsmaterial, des sogenannten Gemisches, stattfindet. Hierzu weist die Erwärmeinrichtung 2 eine Wanne 2a auf, die mit einer Bodenabzugseinrichtung 6a zum Abziehen von Bodenmaterial 11 der Glasschmelze 10 versehen ist.

Um eine ausreichende Homogenität und Blasenfreiheit zu gewährleisten, schließt sich hier dem Einschmelzen nachfolgend die Läuterung der Glasschmelze 10 in einer stromabwärts befindlichen Läutereinrichtung 23 an, die zugleich auch als Transportbereich zum Abtransportieren der Glasschmelze 10 zu einem nachgeordneten Abschnitt eingesetzt werden kann. Ein wesentliches Ziel der Läuterung der Glasschmelze 10 stellt die Entfernung der physikalisch und chemisch in der Glasschmelze 10 gebundenen Gase aus der Glasschmelze 10 dar. Nach Abschluss der Läuterung soll eine Neubildung von Blasen in der Schmelze zumindest reduziert oder gar verhindert werden. Die Läuterung kann grundsätzlich innerhalb derselben Wanne 2a erfolgen oder auch in einer gesonderten Wanne. Der Läutereinrichtung 23 schließt sich eine Konditionierungseinrichtung 3 an, in welchem die Glasschmelze 10 weiter konditioniert und/oder homogenisiert wird. Am Boden der Konditionierungseinrichtung 3 befindet sich eine weitere Bodenabzugseinrichtung 6b.

Die Wände 20 der vorgenannten Einrichtungen 2, 23, 3 der Glasschmelzanlage 1 bestehen aus einem Feuerfestmaterial, wie beispielsweise dem oben beschriebenen schmelzgegossenen Zirkoniumoxidmaterial. Gemäß der Figur 1 ist die Erwärmeinrichtung 2 von der Läutereinrichtung 23 mittels eines strömungsbeeinflussenden Elements 15 getrennt, dass sich quer zur Strömungsrichtung über die gesamte Breite der Glasschmelzanlage 1 in diesem Bereich erstreckt und diesen Bereich im Wesentlichen vollständig versperrt, mit Ausnahme eines geringen Überstands zur Glasstandslinie. Das strömungsbeeinflussende Element 15 kann alternativ oder ergänzend auch im Übergangsbereich zwischen der Läutereinrichtung 23 und der Konditioniereinrichtung 3 angeordnet sein.

Weiterhin weist die Glasschmelzanlage 1 eine Heizeinrichtung 4 mit Elektroden 5 zur Widerstandsbeheizung auf. Diese sind mit einer Wechselstromquelle 8, die Wechselstrom mit einer Frequenz zwischen 1 kHz und 200 kHz als Heizstrom bereitstellt, verbunden. Zusätzlich kann die Glasschmelze z.B. mit Gasbrennern erwärmt werden (nicht gezeigt). Eine Steuereinrichtung 30 steuert die Konditionierungseinrichtung 3, die Erwärmeinrichtung 2, die Wechselstromquelle 8 und ggf. die Läutereinrichtung 23 sowie die Bodenabzugseinrichtungen 6a, 6b derart, dass zum einen Bodenmaterial der Glasschmelze zwischen 1% und 50% des Volumenstroms der Glasschmelze 10 abgezogen wird, beispielsweise 25% und gegebenenfalls wieder der Erwärmeinrichtung 2 zugeführt werden kann, zum anderen, dass eine Widerstandsbeheizung der Glasschmelze 10 zumindest in der Konditionierungseinrichtung 3 erfolgt. Weiterhin weisen die Kontaktflächen 7, die mit der Glasschmelze 10 in Kontakt stehen, Kontaktmaterial 7a in Form von schmelzgegossenem Zirkoniumoxidmaterial mit einem Anteil von über 70 Gew.-% ZrO₂, bevorzugt schmelzgegossenem Zirkoniumoxidmaterial mit einem Anteil von über 85 Gew.-% ZrO₂.

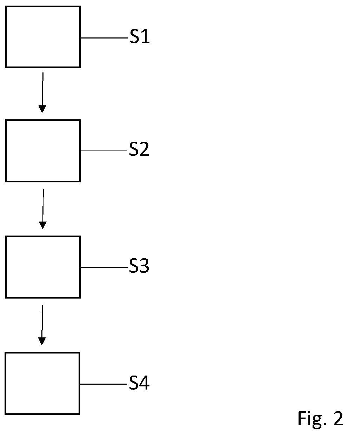

Figur 2 zeigt Schritte eines Verfahrens zur Herstellung eines Glaselements gemäß einer Ausführungsform der vorliegenden Erfindung.

Das Verfahren umfasst die folgenden Schritte:
In einem ersten Schritt S1 erfolgt ein Bereitstellen eines Gemisches gemäß einer Ausführungsform der vorliegenden Erfindung. In einem weiteren Schritt S2 erfolgt ein Erwärmen des Gemisches zu einer Glasschmelze. In einem weiteren Schritt S3 erfolgt ein Konditionieren der Glasschmelze, und in einem weiteren Schritt S4 erfolgt ein Abkühlen der Glasschmelze zur Bereitstellung des Glaselements.

In einer bevorzugten Ausführungsform werden in einem Verfahren gemäß einem der Ansprüche 9 bis 13 zur Herstellung eines Glaselements, bevorzugt in Form einer Glasplatte, bevorzugt einer Borosilikatglasplatte, gemäß einem der Ansprüche 1 bis 12 die folgenden Schritte S1 bis S4; bevorzugt erst S1, dann S2, dann S3 und zuletzt S4; durchgeführt.

### Beispiele

In der folgenden Tabelle wird die Gesamtanzahl der Einschlüsse eines Vergleichsbeispiels und von Beispielen gemäß Ausführungsformen der Erfindung in verschiedenen Kategorien unterteilt in Blasen, nicht-metallische Eischlüsse und metallische Einschlüsse gezeigt:

| Beispiel | Blasen | Nicht-metallische Einschlüsse | Metallische Einschlüsse |
|---|---|---|---|
| [Einheit] | Anzahl/kg | Anzahl/kg | Anzahl/kg |
| 1^{a} | 4,7 | 33,5 | 66,8 |
| 2^{b} | 0,3 | 0,2 | 53,2 |
| 3^{b} | 0 | 0 | 52,4 |
| 4^{b} | 0 | 0 | 8,8 |
| 5^{b} | 0 | 0 | 7,7 |
| 6^{b} | 0 | 0 | 6,4 |

| | | | |
|---|---|---|---|
| a Vergleichsbeispiel b Beispiel gemäß Ausführungsformen der Erfindung | | | |

Die Größenverteilung der metallischen Einschlüsse pro kg Glas von den Beispielen 3 bis 6 im Bereich von 0 bis 50 µm ist in folgender Tabelle dargestellt, wobei nur Einschlüsse mit einer Größe von 2 µm oder mehr im angegebenen Größenintervall 0 µm bis 5 µm der nachfolgenden Tabelle berücksichtigt wurden:

| Beispiel | Größe in µm | | | | | |
|---|---|---|---|---|---|---|
| | 0 bis 5 | 5 bis 10 | 10 bis 20 | 20 bis 30 | 30 bis 40 | 40 bis 50 |
| [Einheit] | Anzahl/kg | Anzahl/kg | Anzahl/kg | Anzahl/kg | Anzahl/kg | Anzahl/kg |
| 3^{b} | 10,5 | 10,5 | 10,5 | 7,0 | 3,5 | 3,5 |
| 4^{b} | 6,9 | 1,9 | 0 | 0 | 0 | 0 |
| 5^{b} | 7,0 | 0,7 | 0 | 0 | 0 | 0 |
| 6^{b} | 5,1 | 1,3 | 0 | 0 | 0 | 0 |

| | | | | | | |
|---|---|---|---|---|---|---|
| b Beispiel gemäß Ausführungsformen der Erfindung | | | | | | |

In Vergleichsbeispiel 1 und Beispiel 2 wurde die Größenverteilung der Einschlüsse nicht genau ermittelt. In Beispielen 2 und 3 war die Gesamtanzahl der Einschlüsse zwar knapp über 50 (z.B. Beispiel 2: 0,3 + 0,2 + 53,2 = 53,7 pro kg) pro kg Glas, jedoch war die Größe einiger Einschlüsse über 50 µm, sodass die Anzahl der Einschlüsse mit einer Größe von 50 µm oder weniger und somit auch die Anzahl der Einschlüsse mit einer Größe von 10 µm oder weniger unter 50 Einschlüssen pro kg Glas lag (siehe zweite Tabelle; Beispiel 3: 10,5 + 10,5 + 10,5 + 7,0 + 3,5 + 3,5 = 45,5 pro kg). Neben den in der zweiten Tabelle angegebenen Einschlüssen wurden in Beispiel 3 noch Einschlüsse gefunden, deren Größe über 100 µm lag. In den Beispielen 3 bis 6 lag die Anzahl der Einschlüsse mit einer Größe von 50 µm oder weniger unter 50 Einschlüssen pro kg (siehe zweite Tabelle); in den Beispielen 4 bis 6 sogar unter 10 Einschlüssen pro kg. Zudem wiesen die Glasplatten der Beispiele 4 bis 6 keine weiteren Einschlüsse mit einer Größe über 50 µm auf (ersichtlich aus Vergleich der ersten und zweiten Tabelle).

Vergleichsbeispiel 1 ist ein Beispiel eines Glaselements in Form einer Glasplatte, bei der keiner der hierin beschriebenen Verbesserungen an der Glasschmelzanlage verwendet wurde, mit anderen Worten eine bisher bekannte Glasschmelzanlage bzw. ein bisher bekanntes Herstellungsverfahren verwendet wurde. Die Gesamtanzahl der Einschlüsse lag hier deutlich über 50 und auch Anzahl der Einschlüsse der jeweiligen Kategorie war sehr hoch. Auch wenn nicht die genaue Größe jedes einzelnen Einschlusses in Vergleichsbeispiel 1 ermittelt wurde, ist davon auszugehen, dass, aufgrund der hohen Anzahl der Einschlüsse auch im Bereich unter 50 µm, die Anzahl der Einschlüsse deutlich über 50 Einschlüsse pro kg liegen muss. Davon ausgehend wurde sukzessiv eine Testglasschmelzanlage immer weiter verbessert.

Als erstes wurde in einem Experiment in der Glasschmelzanlage ein Teil der Kontaktfläche (etwa 70% oder mehr), mit der die Glasschmelze in Kontakt war, mit Kontaktmaterial aus schmelzgegossenem Zirkoniumoxidmaterial mit einem Anteil von über 70 Gew.-% ZrO₂ versehen. Gleichzeitig wurde die Glasschmelzanlage derart betrieben, dass zwischen 1 % und 50 % des Volumenstroms von dem Bodenmaterial der Glasschmelze in einem Bodenbereich der Glasschmelze abgezogen wurde. Dies führte zu einer signifikanten Reduktion der Blasen und nichtmetallischen Einschlüsse (siehe z.B. Beispiel 2). Die Anzahl der metallischen Einschlüsse liegt bei Beispiel 2 über 50 (Anzahl = 53,2), wovon, wie bereits oben beschrieben, zwar ein Großteil der Einschlüsse eine Größe von 50 µm oder weniger aufwies, jedoch auch einige Einschlüsse eine Größe über 50 µm aufwiesen. Somit weist eine derart hergestellte Glasplatte pro kg Glas 50 Einschlüsse oder weniger mit einer Größe von 10 µm oder weniger auf. Bereits ein Versehen eines geringeren Anteils der Kontakt-fläche mit dem speziellen Kontaktmaterial (30 % der Kontakt-fläche oder mehr) führte zu einer Reduktion der Blasen und nicht-metallischen Einschlüsse. 0 Blasen und nicht-metallischen Einschlüsse pro kg mit einer Größe von 50 µm oder weniger wurden bei Glasplatten gefunden, die mit einer Glasschmelzanlage hergestellt wurden, bei der ein Großteil der Kontaktfläche (90% oder mehr oder sogar 95% oder mehr), mit der die Glasschmelze in Kontakt war, schmelzgegossenes Zirkoniumoxidmaterial mit einem hohen Anteil (85 Gew.-% oder mehr, oder sogar 95 Gew.-% oder mehr) an ZrO₂ war, und zudem ein großer Anteil (zwischen 25 Vol.-% und 35 Vol.-%) von dem Bodenmaterial der Glasschmelze in einem Bodenbereich der Glasschmelze abgezogen wurde (siehe Beispiel 3 bis 6).

Ebenfalls wurde die elektrische Widerstandsbeheizung optimiert. Hierzu wurde zu Testzwecken in die Glasschmelzanlage eine neue elektrische Widerstandsbeheizung gemäß einer Ausführungsform der vorliegenden Erfindung eingebaut. In Vergleichsbeispiel 1 wurde eine herkömmliche Widerstandsbeheizung verwendet und eine hohe Anzahl an metallischen Einschlüssen in der damit hergestellten Glasplatte ermittelt. In den Bespielen 2 bis 6 wurde die Glasschmelze zumindest teilweise mit einer elektrischen Widerstandsbeheizung durch Beaufschlagen von Elektroden mit einem Heizstrom beheizt und/oder erwärmt, wobei als Heizstrom ein Wechselstrom mit einer Frequenz zwischen 1 kHz und 200 kHz verwendet wurde, wobei die Anzahl der metallischen Einschlüsse reduziert werden konnte. Die Anzahl der metallischen Einschlüsse konnte weiter deutlich durch Einstellen der Frequenz zwischen 1 kHz und 100 kHz reduziert werden, bis schließlich besonders wenige metallische Einschlüsse bei einer Frequenz zwischen 8 kHz und 15 kHz gefunden wurden (siehe Beispiele 4 bis 6).

Aus den Beispielen ist zudem ersichtlich, dass das beste Ergebnis erzielt wird, wenn alle drei hierin beschriebenen Verbesserungsschritte angewandt werden.

Zusammenfassend weist zumindest eine der Ausführungsformen der Erfindung zumindest einen der folgenden Vorteile auf:
- Reduktion von Einschlüssen, bevorzugt von Einschlüssen von metallischen Partikeln, mit einer Größe von 50 µm oder weniger;
- Erhöhung der Flexibilität des Glases hinsichtlich verschiedener Anwendungen,
- Höhere Qualität des Glases;
- Weniger Ausschuss; und
- Verringerte Anzahl von Bulkdefekten bzw. geringere Bulkdefektdichte.

Das mittels Ausführungsformen der Erfindung hergestellte Glas beziehungsweise Glaselement kann bevorzugt für die folgenden Anwendungen eingesetzt oder verwendet werden:
- Laseranwendungen mit hoher Energiedichte;
- Großformatige, homogene und/oder fehlerarme Fotomasken für lithografische Anwendungen;
- Nanoprägelithografie, Abdruckformen zum Prägen von Präzisionslinsen oder Nanostrukturen bis in den nm-Bereich, bspw. Festplattenmagnetschichten oder dergleichen;
- Substrate für flache Metalinsenkonzepte und -optiken;
- Superglaswafer; und
- Präzisionsfenster in Displays, bevorzugt für OLEDs.

## Patentansprüche

1. Glaselement aus Borosilikatglas,
wobei das Glaselement pro kg Glas 50 Einschlüsse oder weniger mit einer Größe von 2 µm oder mehr und 10 µm oder weniger aufweist, insbesondere wobei das Glaselement pro kg Glas 2000 Einschlüsse oder weniger, bevorzugt 1000 Einschlüsse oder weniger, mehr bevorzugt 500 Einschlüsse oder weniger, mehr bevorzugt 250 Einschlüsse oder weniger, mehr bevorzugt 100 Einschlüsse oder weniger, mehr bevorzugt 50 Einschlüsse oder weniger, mit einer Größe von 500 nm oder mehr und 10 µm oder weniger, bevorzugt mit einer Größe von 50 nm oder mehr und 10 µm oder weniger, mehr bevorzugt von 10 µm oder weniger aufweist.

2. Glaselement gemäß Anspruch 1, wobei das Glaselement pro kg Glas 50 Einschlüsse oder weniger mit einer Größe von 20 µm oder weniger, bevorzugt 30 µm oder weniger, mehr bevorzugt 40 µm oder weniger, meist bevorzugt 50 µm oder weniger und/oder einer Größe von 2 µm oder mehr, bevorzugt 50 nm oder mehr aufweist.

3. Glaselement gemäß einem der Ansprüche 1-2, wobei das Glaselement pro kg Glas 40 Einschlüsse oder weniger, bevorzugt 30 Einschlüsse oder weniger, mehr bevorzugt 20 Einschlüsse oder weniger, meist bevorzugt 10 Einschlüsse oder weniger aufweist.

4. Glaselement gemäß einem der Ansprüche 1 bis 3, wobei die Einschlüsse metallische Einschlüsse umfassen, und wobei das Glaselement pro kg Glas 40 metallische Einschlüsse oder weniger, bevorzugt 30 metallische Einschlüsse oder weniger, mehr bevorzugt 20 metallische Einschlüsse oder weniger, mehr bevorzugt 10 metallische Einschlüsse oder weniger, meist bevorzugt 5 metallische Einschlüsse oder weniger, mit einer Größe von 5 µm oder weniger, bevorzugt 10 µm oder weniger, mehr bevorzugt 20 µm oder weniger, mehr bevorzugt 30 µm oder weniger, mehr bevorzugt 40 µm oder weniger, meist bevorzugt 50 µm oder weniger, aufweist.

5. Glaselement gemäß einem der Ansprüche 1 bis 4, wobei die Einschlüsse nicht-metallische Einschlüsse umfassen, und wobei das Glaselement pro kg Glas 25 nicht-metallische Einschlüsse oder weniger, bevorzugt 15 nicht-metallische Einschlüsse oder weniger, mehr bevorzugt 8 nicht-metallische Einschlüsse oder weniger, mehr bevorzugt 1 nicht-metallische Einschlüsse oder weniger, mehr bevorzugt 0,1 nicht-metallische Einschlüsse oder weniger, mehr bevorzugt 0,05 nicht-metallische Einschlüsse oder weniger, meist bevorzugt 0,00 nicht-metallische Einschlüsse, mit einer Größe von 2 µm oder mehr und 10 µm oder weniger, bevorzugt 2 µm oder mehr und 20 µm oder weniger, mehr bevorzugt 2 µm oder mehr und 30 µm oder weniger, mehr bevorzugt 2 µm oder mehr und 40 µm oder weniger, meist bevorzugt 2 µm oder mehr und 50 µm oder weniger, aufweist.

6. Glaselement gemäß einem der Ansprüche 1 bis 5, wobei die Einschlüsse Blasen umfassen, wobei das Glaselement pro kg Glas 25 Blasen oder weniger, bevorzugt 15 Blasen oder weniger, mehr bevorzugt 8 Blasen oder weniger, mehr bevorzugt 1 Blasen oder weniger, mehr bevorzugt 0,1 Blasen oder weniger, mehr bevorzugt 0,05 Blasen oder weniger, meist bevorzugt 0,00 Blasen, mit einer Größe von 10 µm oder weniger, bevorzugt 20 µm oder weniger, mehr bevorzugt 30 µm oder weniger, mehr bevorzugt 40 µm oder weniger, meist bevorzugt 50 µm oder weniger, und/oder 2 µm oder mehr, bevorzugt 50 nm oder mehr, aufweist.

7. Glaselement gemäß einem der Ansprüche 1 bis 6, wobei die Zusammensetzung des Glaselements umfasst, in Gew.-%:
| | |
|---|---|
| SiO₂ | 60 bis 90 %, bevorzugt 76 % bis 90 %; |
| B₂O₃ | 0 bis 20 %; |
| Al₂O₃ | 0 bis 20 %; |
| | |
|---|---|
| Li₂O | 0 bis 10 %; |
| Na₂O | 0 bis 10 %; |
| K₂O | 0 bis 10 %; |
| MgO | 0 bis 10 %; |
| CaO | 0 bis 10 %; |
| SrO | 0 bis 10 %; und |
| BaO | 0 bis 10 %. |

8. Glaselement gemäß einem der Ansprüche 1 bis 7, wobei das Glaselement ein oder mehrere der folgenden Merkmale aufweist:
i) das Gewicht des Glaselements beträgt 0,01 kg bis 350 kg, bevorzugt 0,1 kg bis 20 kg, mehr bevorzugt 0,5 kg bis 15 kg, meist bevorzugt 1,0 kg bis 10 kg;
ii) die Dicke des Glaselements, wenn dieses in Form einer Glasplatte bereitgestellt ist, beträgt zwischen 0,01 cm und 10 cm, bevorzugt zwischen 0,1 cm und 5 cm, mehr bevorzugt zwischen 0,15 cm und 2,5 cm, meist bevorzugt zwischen 0,2 und 0,4 cm;
iii) die Länge und Breite des Glaselements, wenn dieses in Form einer Glasplatte bereitgestellt ist, beträgt jeweils zwischen 1 cm und 500 cm, bevorzugt zwischen 3 cm und 400 cm, mehr bevorzugt zwischen 5 und 100 cm, mehr bevorzugt zwischen 20 und 70 cm, meist bevorzugt 40 cm bis 60 cm;
iv) die Transmission des Glaselements normiert auf ein Glaselement in Form einer Glasplatte mit einer Dicke von 6,5 mm bei einer Wellenlänge von 400 nm bis 800 nm beträgt 70 % oder mehr, mehr bevorzugt 80% oder mehr, mehr bevorzugt 90 % oder mehr, meist bevorzugt 95 % oder mehr; und
v) das Glaselement weist keine Einschlüsse mit einer Größe von mehr als 50 µm, bevorzugt mehr als 40 µm, mehr bevorzugt mehr als 30 µm, mehr bevorzugt mehr als 20 µm, meist bevorzugt mehr als 10 µm, auf.

9. Glaselement gemäß einem der Ansprüche 1-8, wobei das Glaselement in Form einer Glasplatte oder eines Glasrohrs bereitgestellt ist.

10. Glaselement gemäß einem der Ansprüche 1-9, wobei das Glaselement pro kg Glas 2000 Einschlüsse oder weniger, bevorzugt 1000 Einschlüsse oder weniger, mehr bevorzugt 500 Einschlüsse oder weniger, mehr bevorzugt 250 Einschlüsse oder weniger, mehr bevorzugt 100 Einschlüsse oder weniger, mehr bevorzugt 50 Einschlüsse oder weniger mit einer Größe von 50 nm oder mehr und 500 nm oder weniger aufweist und/oder wobei
das Glaselement pro kg Glas 2000 Einschlüsse oder weniger, bevorzugt 1000 Einschlüsse oder weniger, mehr bevorzugt 500 Einschlüsse oder weniger, mehr bevorzugt 250 Einschlüsse oder weniger, mehr bevorzugt 100 Einschlüsse oder weniger, mehr bevorzugt 50 Einschlüsse oder weniger mit einer Größe von 500 nm oder mehr und 2 µm oder weniger aufweist.

11. Verfahren zur Herstellung eines Glaselements aus Borosilikatglas, gemäß einem der Ansprüche 1 bis 10, umfassend die Schritte:
i) Bereitstellen (S1) eines Gemisches, umfassend, in Gew.-%:
| | |
|---|---|
| SiO₂ | 60 bis 90 %, bevorzugt 76 % bis 90 %; |
| B₂O₃ | 0 bis 20 %; |
| Al₂O₃ | 0 bis 20 %; |
| Li₂O | 0 bis 10 %; |
| Na₂O | 0 bis 10 %; |
| K₂O | 0 bis 10 %; |
| MgO | 0 bis 10 %; |
| CaO | 0 bis 10 %; |
| SrO | 0 bis 10 %; und |
| BaO | 0 bis 10 %; |
ii) Erwärmen (S2) des Gemisches zu einer Glasschmelze (10),
iii) Konditionieren (S3) der Glasschmelze (10), und
iv) Abkühlen (S4) der Glasschmelze (10) zur Bereitstellung des Glaselements,
wobei die Kontaktfläche (7), mit der die Glasschmelze (10) in Kontakt ist, zu 30 % oder mehr Kontaktmaterial (7a) in Form von schmelzgegossenem Zirkoniumoxidmaterial mit einem Anteil von über 70 Gew.-% ZrO₂ umfasst und insbesondere wobei zumindest eine, bevorzugt alle, der folgenden Bedingungen a) und c) während der Schritte ii) und/oder iii) erfüllt wird:
a) die Glasschmelze (10) wird zumindest teilweise mit einer elektrischen Widerstandsbeheizung durch Beaufschlagen von Elektroden (5) mit einem Heizstrom beheizt und/oder erwärmt, wobei als Heizstrom ein Wechselstrom mit einer Frequenz zwischen 1 kHz und 200 kHz verwendet wird; und
c) zwischen 1 % und 50 % des Volumenstroms von dem Bodenmaterial (11) der Glasschmelze (10) wird in einem Bodenbereich der Glasschmelze (10) abgezogen,
insbesondere wobei
die Viskosität der Glasschmelze (10) zumindest in den Schritten ii) und/oder iii) auf einen Wert zwischen 30 Pas und 450 Pas, bevorzugt zwischen 33 Pas und 400 Pas, mehr bevorzugt zwischen 35 Pas und 265 Pas gebracht wird und/oder der Wert gehalten wird.

12. Verfahren gemäß Anspruch 11, wobei als Heizstrom ein Wechselstrom mit einer Frequenz zwischen 1 kHz und 100 kHz, bevorzugt zwischen 5 kHz und 50 kHz, mehr bevorzugt zwischen 8 kHz und 15 kHz, verwendet wird und/oder wobei
die Kontaktfläche (7), mit der die Glasschmelze (10) in Kontakt ist, zu 60 % oder mehr, bevorzugt 80 % oder mehr, mehr bevorzugt 90 % oder mehr, meist bevorzugt 95% oder mehr Kontaktmaterial (7a) in Form von schmelzgegossenem Zirkoniumoxidmaterial mit einem Anteil von 75 Gew.-% oder mehr; bevorzugt 85 Gew.-% oder mehr, mehr bevorzugt 95 Gew.-% oder mehr ZrO₂ umfasst und/oder wobei
zwischen 5 % und 40 %, bevorzugt zwischen 20 % und 40 %, mehr bevorzugt zwischen 25 % und 35 %, des Volumenstroms von dem Bodenmaterial (11) der Glasschmelze (10) in einem Bodenbereich abgezogen wird.

13. Glasschmelzanlage (1) zur Durchführung eines Verfahrens gemäß einem der Ansprüche 11 bis 12, umfassend
eine Erwärmeinrichtung (2) zum Erwärmen eines Gemisches zu einer Glasschmelze (10),
eine Konditionierungseinrichtung (3) zum Konditionieren der Glasschmelze (10), und eine Bodenabzugseinrichtung (6a, 6b) zum Abziehen von Bodenmaterial (11) der Glasschmelze (10),
wobei die Erwärmeinrichtung (2) und/oder die Konditionierungseinrichtung (3) eine Heizeinrichtung (4) mit zumindest zwei Elektroden zur Widerstandsbeheizung umfasst; und wobei die Kontaktfläche (7), mit der die Glasschmelze (10) in Kontakt ist, derart ausgebildet ist, dass sie zu 30 % oder mehr Kontaktmaterial (7a) in Form von schmelzgegossenem Zirkoniumoxidmaterial mit einem Anteil von über 70 Gew.-% ZrO₂, bevorzugt schmelzgegossenes Zirkoniumoxidmaterial mit einem Anteil von über 85 Gew.-% ZrO₂, umfasst,
und insbesondere wobei zumindest eine der Bedingungen A) und C) erfüllt ist:
A) die Heizeinrichtung (4) derart ausgebildet oder gesteuert ist, dass die zumindest zwei Elektroden (5) mit einem Heizstrom in Form eines Wechselstroms mit einer Frequenz zwischen 1 kHz und 200 kHz beaufschlagt werden; und/ oder
C) die Bodenabzugseinrichtung (6a, 6b) derart ausgebildet ist, zwischen 1 % und 50 % des Volumenstroms von dem Bodenmaterial (11) der Glasschmelze (10) in einem Bodenbereich von Erwärmeinrichtung (2) und/oder Konditionierungseinrichtung (3) abzuziehen.

14. Verfahren zum Betreiben einer Glasschmelzanlage (1) gemäß Anspruch 13, wobei zumindest Schritte I) und II) oder Schritte II) und III) oder Schritte I), II) und III) durchgeführt werden:
I) Betreiben der Heizeinrichtung (4) durch Beaufschlagen der Elektroden (5) mit einem Heizstrom, wobei als Heizstrom ein Wechselstrom mit einer Frequenz zwischen 1 kHz und 200 kHz verwendet wird,
II) In Kontakt bringen der Glasschmelze (10) mit einer Kontaktfläche (7), mit der die Glasschmelze (10) in Kontakt ist, die zu 30 % oder mehr schmelzgegossenes Zirkoniumoxidmaterial mit einem Anteil von über 70 Gew.-% ZrO₂, bevorzugt schmelzgegossenes Zirkoniumoxidmaterial mit einem Anteil von über 85 Gew.-% ZrO₂, umfasst; und
III) Betreiben der Bodenabzugseinrichtung (6a, 6b) derart, dass zwischen 1 % und 50 % des Volumenstroms von dem Bodenmaterial (11) der Glasschmelze (10) in einem Bodenbereich abgezogen wird.

## Claims

1. Glass element made of borosilicate glass, wherein the glass element has per kg of glass 50 inclusions or less having a size of 2 µm or more and 10 µm or less, in particular wherein the glass element has per kg of glass 2000 inclusions or less, preferably 1000 inclusions or less, more preferably 500 inclusions or less, more preferably 250 inclusions or less, more preferably 100 inclusions or less, more preferably 50 inclusions or less, having a size of 500 nm or more and 10 µm or less, preferably having a size of 50 nm or more and 10 µm or less, more preferably of 10 µm or less.

2. Glass element according to claim 1, wherein the glass element has per kg of glass 50 inclusions or less having a size of 20 µm or less, preferably 30 µm or less, more preferably 40 µm or less, most preferably 50 µm or less and/or a size of 2 µm or more, preferably 50 nm or more.

3. Glass element according to any one of claims 1 to 2, wherein the glass element has per kg of glass 40 inclusions or less, preferably 30 inclusions or less, more preferably 20 inclusions or less, most preferably 10 inclusions or less.

4. Glass element according to any one of claims 1 to 3, wherein the inclusions comprise metal inclusions, and wherein the glass element has per kg of glass 40 metal inclusions or less, preferably 30 metal inclusions or less, more preferably 20 metal inclusions or less, more preferably 10 metal inclusions or less, most preferably 5 metal inclusions or less, having a size of 5 µm or less, preferably 10 µm or less, more preferably 20 µm or less, more preferably 30 µm or less, more preferably 40 µm or less, most preferably 50 µm or less.

5. Glass element according to any one of claims 1 to 4, wherein the inclusions comprise non-metal inclusions, and wherein the glass element has per kg of glass 25 non-metal inclusions or less, preferably 15 non-metal inclusions or less, more preferably 8 non-metal inclusions or less, more preferably 1 non-metal inclusion or less, more preferably 0.1 non-metal inclusions or less, more preferably 0.05 non-metal inclusions or less, most preferably 0.00 non-metal inclusions, having a size of 2 µm or more and 10 µm or less, preferably 2 µm or more and 20 µm or less, more preferably 2 µm or more and 30 µm or less, more preferably 2 µm or more and 40 µm or less, most preferably 2 µm or more and 50 µm or less.

6. Glass element according to any one of claims 1 to 5, wherein the inclusions comprise bubbles, wherein the glass element has per kg of glass 25 bubbles or less, preferably 15 bubbles or less, more preferably 8 bubbles or less, more preferably 1 bubble or less, more preferably 0.1 bubbles or less, more preferably 0.05 bubbles or less, most preferably 0.00 bubbles, having a size of 10 µm or less, preferably 20 µm or less, more preferably 30 µm or less, more preferably 40 µm or less, most preferably 50 µm or less, and/or 2 µm or more, preferably 50 nm or more.

7. Glass element according to any one of claims 1 to 6, wherein the composition of the glass element comprises in % by weight:
SiO₂ 60 to 90%, preferably 76% to 90%;
B₂O₃ 0 to 20%;
Al₂O₃ 0 to 20%;
Li₂O 0 to 10%;
Na₂O 0 to 10%;
K₂O 0 to 10%;
MgO 0 to 10%;
CaO 0 to 10%;
SrO 0 to 10%; and
BaO 0 to 10%.

8. Glass element according to any one of claims 1 to 7, wherein the glass element has one or more of the following features:
i) the weight of the glass element is from 0.01 kg to 350 kg, preferably 0.1 kg to 20 kg, more preferably 0.5 kg to 15 kg, most preferably 1.0 kg to 10 kg;
ii) the thickness of the glass element, when it is provided in the form of a glass plate, is between 0.01 cm and 10 cm, preferably between 0.1 cm and 5 cm, more preferably between 0.15 cm and 2.5 cm, most preferably between 0.2 cm and 0.4 cm;
iii) the length and width of the glass element, when it is provided in the form of a glass plate, is in each case between 1 cm and 500 cm, preferably between 3 cm and 400 cm, more preferably between 5 and 100 cm, more preferably between 20 and 70 cm, most preferably from 40 cm to 60 cm;
iv) the transmission of the glass element standardised to a glass element in the form of a glass plate having a thickness of 6.5 mm at a wavelength of from 400 nm to 800 nm is 70% or more, more preferably 80% or more, more preferably 90% or more, most preferably 95% or more; and
v) the glass element has no inclusions having a size of more than 50 µm, preferably more than 40 µm, more preferably more than 30 µm, more preferably more than 20 µm, most preferably more than 10 µm.

9. Glass element according to any one of claims 1 to 8, wherein the glass element is provided in the form of a glass plate or a glass pipe.

10. Glass element according to any one of claims 1 to 9,
wherein the glass element has per kg of glass 2000 inclusions or less, preferably 1000 inclusions or less, more preferably 500 inclusions or less, more preferably 250 inclusions or less, more preferably 100 inclusions or less, more preferably 50 inclusions or less having a size of 50 nm or more and 500 nm or less and/or wherein
the glass element has per kg of glass 2000 inclusions or less, preferably 1000 inclusions or less, more preferably 500 inclusions or less, more preferably 250 inclusions or less, more preferably 100 inclusions or less, more preferably 50 inclusions or less having a size of 500 nm or more and 2 µm or less.

11. Method for producing a glass element from boron silicate glass according to any one of claims 1 to 10, comprising the steps of:
i) providing (S1) an admixture comprising in % by weight:
SiO₂ 60 to 90%, preferably 76% to 90%;
B₂O₃ 0 to 20%;
Al₂O₃ 0 to 20%;
Li₂O 0 to 10%;
Na₂O 0 to 10%;
K₂O 0 to 10%;
MgO 0 to 10%;
CaO 0 to 10%;
SrO 0 to 10%; and
BaO 0 to 10%;
ii) heating (S2) the admixture to form a glass melt (10),
iii) conditioning (S3) the glass melt (10), and
iv) cooling (S4) the glass melt (10) to provide the glass element, wherein the contact face (7) with which the glass melt (10) is in contact comprises at a rate of 30% or more contact material (7a) in the form of fusion cast zirconium oxide material having a proportion of over 70% by weight ZrO₂ and in particular wherein at least one, preferably all of the following conditions a) and c) is/are complied with during the steps ii) and/or iii):
a) the glass melt (10) is at least partially heated and/or warmed with an electrical resistance heating by acting on electrodes (5) with a heating current, wherein as the heating current an alternating current is used at a frequency between 1 kHz and 200 kHz; and
c) between 1% and 50% of the volume flow of the base material (11) of the glass metal (10) is removed in a base region of the glass melt (10),
in particular wherein
the viscosity of the glass melt (10) at least in the steps ii) and/or iii) is brought to a value between 30 Pas and 450 Pas, preferably between 33 Pas and 400 Pas, more preferably between 35 Pas and 265 Pas and/or the value is maintained.

12. Method according to claim 11, wherein as the heating current an alternating current at a frequency between 1 kHz and 100 kHz, preferably between 5 kHz and 50 kHz, more preferably between 8 kHz and 15 kHz is used and/or wherein the contact face (7) with which the glass melt (10) is in contact comprises at a rate of 60% or more, preferably 80% or more, more preferably 90% or more, most preferably 95% or more contact material (7a) in the form of fusion cast zirconium oxide material having a proportion of 75% by weight or more, preferably 85% by weight or more, more preferably 95% by weight or more ZrO₂ and/or wherein between 5% and 40%, preferably between 20% and 40%, more preferably between 25% and 35% of the volume flow of the base material (11) of the glass melt (10) is removed in a base region.

13. Glass melt installation (1) for carrying out a method according to any one of claims 11 to 12, comprising:
a heating device (2) for heating an admixture to form a glass melt (10),
a conditioning device (3) for conditioning the glass melt (10), and a base removal device (6a, 6b) for removing base material (11) of the glass melt (10),
wherein the heating device (2) and/or the conditioning device (3) comprises a heating device (4) having at least two electrodes for resistance heating; and wherein the contact face (7) with which the glass melt (10) is in contact is constructed in such a manner that it comprises at a rate of 30% or more contact material (7a) in the form of fusion cast zirconium oxide material having a proportion of over 70% by weight ZrO₂, preferably fusion cast zirconium oxide material having a proportion of over 85% by weight ZrO₂,
and in particular wherein at least one of the conditions A) and C) is complied with:
A) the heating device (4) is constructed or controlled in such a manner that the at least two electrodes (5) are acted on with a heating current in the form of an alternating current at a frequency between 1 kHz and 200 kHz; and/or
C) the base removal device (6a, 6b) is constructed to remove between 1% and 50% of the volume flow from the base material (11) of the glass melt (10) in a base region of the heating device (2) and/or conditioning device (3).

14. Method for operating a glass melt installation (1) according to claim 13, wherein at least steps I) and II) or steps II) and III) or steps I), II) and III) are carried out:
I) operating the heating device (4) by acting on the electrodes (5) with a heating current, wherein as the heating current an alternating current is used at a frequency between 1 kHz and 200 kHz,
II) bringing the glass melt (10) into contact with a contact face (7) with which the glass melt (10) is in contact and which comprises at a rate of 30% or more fusion cast zirconium oxide material having a proportion of over 70% by weight ZrO₂, preferably fusion cast zirconium oxide material having a proportion of over 85% by weight ZrO₂; and
III) operating the base removal device (6a, 6b) in such a manner that between 1% and 50% of the volume flow is removed from the base material (11) of the glass melt (10) in a base region.

## Revendications

1. Élément de verre en verre borosilicate,
dans lequel l'élément de verre comprend, par kg de verre, 50 inclusions ou moins avec une taille de 2 µm ou plus et 10 µm ou moins, plus particulièrement dans lequel l'élément de verre comprend, par kg de verre, 2000 inclusions ou moins, de préférence 1000 inclusions ou moins, plus de préférence 500 inclusions ou moins, plus de préférence 250 inclusions ou moins, plus de préférence 100 inclusions ou moins, plus de préférence 50 inclusions ou moins, avec une taille de 500 nm ou plus et de 10 µm ou moins, de préférence avec une taille de 50 nm ou plus et de 10 µm ou moins, plus de préférence de 10 µm ou moins.

2. Élément selon la revendication 1, dans lequel l'élément de verre comprend, par kg de verre, 50 inclusions ou moins avec une taille de 20 µm ou moins, de préférence 30 µm ou moins, plus de préférence 40 µm ou moins, le plus de préférence 50 µm ou moins et/ou une taille de 2 µm ou plus, de préférence 50 nm ou plus.

3. Élément selon l'une des revendications 1 à 2, dans lequel l'élément de verre comprend, par kg, 40 inclusions ou moins, de préférence 30 inclusions ou moins, plus de préférence 20 inclusions ou moins, le plus de préférence 10 inclusions ou moins.

4. Élément selon l'une des revendications 1 à 3, dans lequel les inclusions comprennent des inclusions métalliques et dans lequel l'élément de verre comprend, par kg de verre, 40 inclusions métalliques ou moins, de préférence 30 inclusions métalliques au moins, plus de préférence 20 inclusions métalliques au moins, plus de préférence 10 inclusions métalliques ou moins, le plus de préférence 5 inclusions métalliques ou moins, avec une taille de 5 µm ou moins, de préférence 10 µm ou moins, plus de préférence 20 µm ou moins, plus de préférence 30 µm ou moins, plus de préférence 40 µm ou moins, le plus de préférence 50 µm ou moins.

5. Élément selon l'une des revendications 1 à 4, dans lequel les inclusions comprennent des inclusions non métalliques et dans lequel l'élément de verre comprend, par kg de verre, 25 inclusions non métalliques ou moins, de préférence 15 inclusions non métalliques au moins, plus de préférence 8 inclusions non métalliques au moins, plus de préférence 1 inclusion non métallique ou moins, plus de préférence 0,1 inclusion non métallique ou moins, plus de préférence 0,05 inclusion non métallique ou moins, le plus de préférence 0,00 inclusion non métallique ou moins, avec une taille de 2 µm ou plus et de 10 µm ou moins, de préférence 2 µm ou plus et 20 µm ou moins, plus de préférence de 2 µm ou plus et 30 µm ou moins, plus de préférence 2 µm ou plus et 40 µm ou moins, le plus de préférence 2 µm ou plus et 50 µm ou moins.

6. Élément selon l'une des revendications 1 à 5, dans lequel les inclusions comprennent des bulles, dans lequel l'élément de verre comprend, par kg de verre, 25 bulles ou moins, de préférence 15 bulles ou moins, plus de préférence 8 bulles ou moins, plus de préférence 1 bulle ou moins, plus de préférence 0,1 bulles ou moins, plus de préférence 0,05 bulle ou moins, le plus de préférence 0,00 bulle ou moins, avec une taille de 10 µm ou moins, de préférence 20 µm ou moins, plus de préférence 30 µm ou moins, plus de préférence 40 µm ou moins, le plus de préférence 50 µm ou moins et/ou 2 µm ou plus, de préférence 50 nm ou plus.

7. Élément selon l'une des revendications 1 à 6, dans lequel la composition de l'élément de verre comprend, en % en poids :
| | |
|---|---|
| SiO₂ | 60 à 90 %, de préférence 76 % à 90 % ; |
| B₂O₃ | 0 à 20 % ; |
| Al₂O₃ | 0 à 20 % ; |
| Li₂O | 0 à 10 % ; |
| Na₂O | 0 à 10 % ; |
| K₂O | 0 à 10 % ; |
| MgO | 0 à 10 % ; |
| CaO | 0 à 10 % ; |
| SrO | 0 à 10 % ; et |
| BaO | 0 à 10 %. |

8. Élément selon l'une des revendications 1 à 7, dans lequel l'élément de verre présente une ou plusieurs des caractéristiques suivantes :
i) le poids de l'élément de verre est égal à 0,01 kg à 350 kg, de préférence 0,1 kg à 20 kg, plus de préférence 0,5 kg à 15 kg, le plus de préférence 1,0 kg à 10 kg ;
ii) l'épaisseur de l'élément de verre, lorsque celui-ci se présente sous la forme d'une plaque de verre, est entre 0,01 cm et 10 cm, de préférence entre 0,1 cm et 5 cm, plus de préférence entre 0,15 cm et 2,5 cm, le plus de préférence entre 0,2 et 0,4 cm ;
iii) la longueur et la largeur de l'élément de verre, lorsque celui-ci se présente sous al forme d'une plaque de verre, est respectivement entre 1 cm et 500 cm, de préférence entre 3 cm et 400 cm, plus de préférence entre 5 et 100 cm, plus de préférence entre 20 et 70 cm, le plus de préférence de 40 cm à 60 cm ;
iv) le taux de transmission de l'élément de verre, normalisé sur un élément de verre sous la forme d'une plaque de verre avec une épaisseur de 6,5 mm, pour une longueur d'onde de 400 nm à 800 nm, est égal à 70 % ou plus, plus de préférence 80 % ou plus, plus de préférence 90 % ou plus, le plus de préférence 95 % ou plus et
v) l'élément de verre ne comprend aucune inclusion avec une taille supérieure à 50 µm, de préférence supérieure à 40 µm, plus de préférence supérieure à 30 µm, plus de préférence supérieure à 20 µm, le plus de préférence supérieure à 10 µm.

9. Élément de verre selon l'une des revendications 1 à 8, dans lequel l'élément de verre se présente sous la forme d'une plaque de verre ou d'un tube de verre.

10. Élément de verre selon l'une des revendications 1 à 9, dans lequel l'élément de verre comprend, par kg de verre, 2000 inclusions ou moins, de préférence 1000 inclusions ou moins, plus de préférence 500 inclusions ou moins, plus de préférence 250 inclusions ou moins, plus de préférence 100 inclusions ou moins, plus de préférence 50 inclusions ou moins avec une taille de 50 nm ou plus et 500 nm ou moins et/ou dans lequel
l'élément de verre comprend, par kg de verre, 2000 inclusions ou moins, de préférence 1000 inclusions ou moins, plus de préférence 500 inclusions ou moins, plus de préférence 250 inclusions ou moins, plus de préférence 100 inclusions ou moins, plus de préférence 50 inclusions ou moins avec une taille de 500 nm ou plus et 2 µm ou moins.

11. Procédé de fabrication d'un élément de verre en verre borosilicate, selon l'une des revendications 1 à 10, comprenant les étapes :
i) mise à disposition (S1) d'un mélange comprenant, en % en poids :
| | |
|---|---|
| SiO₂ | 60 à 90 %, de préférence 76 % à 90 % ; |
| B₂O₃ | 0 à 20 % ; |
| Al₂O₃ | 0 à 20 % ; |
| Li₂O | 0 à 10 % ; |
| Na₂O | 0 à 10 % ; |
| K₂O | 0 à 10 % ; |
| MgO | 0 à 10 % ; |
| CaO | 0 à 10 % ; |
| SrO | 0 à 10 % ; et |
| BaO | 0 à 10 % ; |
ii) chauffage (S2) du mélange afin d'obtenir une fonte de verre (10),
iii) conditionnement (S3) d'une fonte de verre (10) et
iv) refroidissement (S4) de la fonte de verre (10) afin de créer l'élément de verre,
dans lequel la surface de contact (7), avec laquelle la fonte de verre (10) est en contact, comprend, à hauteur de 30 % ou plus, un matériau de contact (7a) sous la forme d'un matériau de type oxyde de zirconium coulé avec une part supérieure de 70 % en poids de ZrO₂ et plus particulièrement dans lequel au moins une, de préférence toutes les conditions suivantes a) et c) sont remplies pendant les étapes ii) et/ou iii) :
a) la fonte de verre (10) est d'abord chauffée au moins partiellement avec un chauffage électrique à résistance par l'alimentation d'électrodes (5) avec un courant de chauffage, dans lequel, en tant que courant de chauffage, on utilise un courant alternatif avec une fréquence entre 1 kHz et 200 kHz ; et
c) entre 1 % et 50 % du flux volumique du matériau de contact (11) de la fonte de verre (10) est extrait dans une zone de fond de la fonte de verre (10),
plus particulièrement dans lequel
la viscosité de la fonte de verre (10) est maintenue, au moins dans les étapes ii) et/ou iii), à une valeur entre 30 Pas et 450 Pas, de préférence entre 33 Pas et 400 Pas, plus de préférence entre 35 Pas et 265 Pas et/ou la valeur.

12. Procédé selon la revendication 11, dans lequel le courant de chauffage utilisé est un courant alternatif avec une fréquence entre 1 kHz et 100 kHz, de préférence entre 5 kHz et 50 kHz, plus de préférence entre 8 kHz et 15 kHz et/ou dans lequel la surface de contact (7), avec laquelle la fonte de verre (10) est en contact, comprend, à hauteur de 60 % ou plus, de préférence 80 % ou plus, plus de préférence 90 % ou plus, le plus de préférence 95 % ou plus, un matériau de contact (7a) sous la forme d'un matériau de type oxyde de zirconium coulé, avec une part de 75 % en poids ou plus, de préférence 85 % en poids ou plus, plus de préférence 95 % en poids ou plus de ZrO₂ et/ou dans lequel
entre 5 % et 40 %, de préférence entre 20 % et 40 %, plus de préférence entre 25 % et 35 % du flux volumique est extrait du matériau de fond (11) de la fonte de verre (10) dans une zone de fond.

13. Installation de fonte de verre (1) pour l'exécution d'un procédé selon l'une des revendications 11 à 12, comprenant
un dispositif de chauffage (2) pour le chauffage d'un mélange afin d'obtenir une fonte de verre (10),
un dispositif de conditionnement (3) pour le conditionnement de la fonte de verre (10) et un dispositif d'extraction de fond (6a, 6b) pour l'extraction d'un matériau de fond (11) de la fonte de verre (10),
dans lequel le dispositif de chauffage (2) et/ou le dispositif de conditionnement (3) comprend un dispositif de chauffage (4) avec au moins deux électrodes pour un chauffage par résistance et dans lequel la surface de contact (7), avec laquelle la fonte de verre (10) est en contact, est conçue de sorte qu'elle comprend, à hauteur de 30 % ou plus, un matériau de contact (7a) sous la forme d'un matériau de type oxyde de zirconium coulé, avec une part supérieure à 70 % en poids de ZrO₂, de préférence un matériau de type oxyde de zirconium coulé avec une part supérieure à 85 % en poids de ZrO₂,
et plus particulièrement dans lequel au moins une des conditions A et C) est remplie :
A) le dispositif de chauffage (4) est conçu et contrôlé de sorte que les au moins deux électrodes (5) sont alimentées avec un courant de chauffage sous la forme d'un courant alternatif avec une fréquence entre 1 kHz et 200 kHz ; et/ou
C) le dispositif d'extraction de fond (6a, 6b) est conçu pour extraire entre 1 % et 50 % du flux volumique du matériau de fond (11) de la fonte de verre (10) dans une zone de fond du dispositif de chauffage (2) et/ou du dispositif de conditionnement (3),

14. Procédé de fonctionnement d'une installation de fonte de verre (1) selon la revendication 13, dans lequel au moins les étapes I) et II) ou les étapes II) et II) ou les étapes I), II) et III) sont exécutées :
I) fonctionnement du dispositif de chauffage (4) par l'alimentation des électrodes (5) avec un courant de chauffage, dans lequel on utilise un courant de chauffage avec une fréquence entre 1 kHz et 200 kHz,
II) mise en contact de la fonte de verre (10) avec une surface de contact (7), avec laquelle la fonte de verre (10) est en contact, qui comprend, à hauteur de 30 % ou plus, un matériau de type oxyde de zirconium coulé, avec une part supérieure à 70 % en poids de ZrO₂, de préférence un matériau de type oxyde de zirconium coulé, avec une part supérieure à 85 % en poids de ZrO₂ ; et
III) fonctionnement du dispositif d'extraction de fond (6a, 6b) de sorte que, entre 1 % et 50 % du flux volumique du matériau de fond (11) de la fonte de verre (10) est extrait dans une zone de fond.
